# EUROPEAN PATENT APPLICATION

(11) **EP 4 800 254 A2**
(43) Date of publication of application: **02.09.2026**
(21) Application number: 26193691.8
(22) Date of filing: 01.11.2024
(51) Int. Cl.: F16B 19/06

(54) **IMPROVEMENTS IN AND RELATING TO THE MANUFACTURE OF HANDHELD JOINTED INSTRUMENTS**

(30) Priority: 03.11.2023 GB 202316925
(62) Document number(s) of the earlier application(s) in accordance with Art. 76 EPC: 24804931.4 / 4 784 012
(71) Applicant: Endomagnetics LTD, Cambridge Cambridgeshire CB4 0WN (GB)
(72) Inventor: PROFIT, Alan Lynford, Letchworth Garden City (GB)
(74) Representative: Abel & Imray LLP

(57) **Abstract**

A surgical tool (1) comprising: a plurality of arms (2), each of the plurality of arms comprises an aperture; an elongate pin extending through the aperture of each of the plurality of arms, said elongate pin (8) comprising an end stop (10) at a first end of the elongate pin, said stop (10) being adjacent to the aperture of a first arm (3) of the plurality of arms (2), and an end portion at a second end of the elongate pin, said end portion extending beyond a second arm (4) of the plurality of arms; a bearing plate mounted onto said end portion of the pin directly adjacent to the aperture of the second arm (4), wherein the end portion comprises a deformed region, deformed such that the pin (8) forms an interference fit with the bearing plate. Also provided is a method (400) of manufacturing said surgical tool.

## Description

### Field of the Disclosure

The present disclosure relates to surgical tools, for example for tools used in various open surgeries. For example, the tools may be used to grasp, hold or manipulate tissues and objects as part of surgical operations concerning the removal of tumours, lesions and other abnormalities, which can be localised using a hand-held probe that emits an oscillating magnetic field to detect a marker previously inserted in or near the tumour, lesion or other abnormality.

### Background

With the increasing prevalence of mammography screening programmes, the majority of breast cancers are detected as small, non-palpable (or occult) lesions in the upper, outer quadrant of the breast, which are amenable to breast conserving treatment. Accurate localisation helps to avoid excision of excess breast tissue which could result in adverse cosmetic results. Accurate localisation is often also required in the treatment of other cancers such for example as colorectal, prostate and lung cancer, as well as other conditions known by those of skilled in the art.

US 2019/0029560 A1 (Endomagnetics Ltd), for example, discloses a magnetic marker comprising a single plug made of a single magnetically detectable ferromagnetic material, which has a magnetic susceptibility such that the marker is detectable using a handheld magnetic susceptometry probe. Meanwhile, WO 2011/067576 A1 (Endomagnetics Ltd) discloses a system and method for locating injectable magnetic nanoparticles having a mean hydrodynamic diameter of 5-200 nm and preferably between 10-50 nm. Suitable susceptometry probes are disclosed by US 8174259 B2 (Hattersley et al.), WO 2014/140566 A1 (Endomagnetics Ltd) and US 9239314 B2 (Endomagnetics Ltd and University of Houston), the contents of all of which are incorporated herein by reference.

Given that the magnetic markers listed above are located with the use of very sensitive detection equipment, there is a need for tools for use in surgeries involving such markers that are non-metallic, because metallic tools may interfere with the accurate detection of the markers. The tools should therefore be made of a non-metallic material.

Moreover, there are environmental and economic benefits of tools being re-useable. For tools to be reusable, it is necessary for them to be sufficiently easy to sterilise.

The tools should also preferably be ergonomic and have a feeling in use comparable to metallic tools, as surgeons are more used to handling metallic tools.

There is also a need for improved methods of joining moving parts of tools such that reliable and low friction, metal-free joints can be achieved which will not induce excessive wear on the parts of the tool in use, such that the longevity of the tool is improved.

Thus, there is an unmet need for surgical tools which do not interact with magnetic fields, are easy to clean and ergonomic to use, and which have a longevity and feel in use which are comparable to those of metallic tools, as well as methods of manufacturing tools that have these attributes.

### Summary of the Disclosure

In accordance with a first aspect of the present disclosure therefore there is provided a surgical tool comprising a plurality of arms, wherein each of the plurality of arms defines an aperture, an elongate pin extending through the aperture of each of the plurality of arms, said elongate pin having an end stop at a first end of the elongate pin, said end stop being adjacent to the aperture of a first arm of the plurality of arms, and an end portion at a second end of the elongate pin, said end portion extending beyond a second arm of the plurality of arms, a bearing plate mounted on said end portion of the pin directly adjacent to the aperture of the second arm, the bearing plate being configured to rotate freely relative to the second arm, wherein the end portion comprises a deformed region, deformed such that the pin forms an interference fit with the bearing plate, thereby pivotally mounting the plurality of arms relative to each other such that at least one of plurality of arms can freely rotate about the elongate pin.

It is well known in the art that the term "interference fit" refers to a physical or mechanical bond between two components, and does not imply a bond formed by polymer in components melting and mixing (e.g., a bond formed by polymer melting being a "fusion bond" formed by mixing of the polymer of the bearing plate and elongate pin when the polymer melts) or a chemical bond (e.g., a bond formed by an adhesive). Thus, for the avoidance of doubt and by way of example, use of adhesives or the like, or polymer welding technologies in the joining of the bearing plate and elongate pin would not be considered to form an interference fit in accordance with the present disclosure.

It may be that the elongate pin is integrally formed with one of the plurality of arms. It may be that the elongate pin is a component separate from each of the plurality of arms. Preferably, each of the plurality of arms can freely rotate about the elongate pin.

A surgical tool as described above joins the plurality of arms in such a way that very little stress is applied to the join in use, as at least one of, and preferably at least two of, the plurality of arms can freely rotate with respect to the elongate pin. Thus, in such an arrangement there is no need for mounting of the arm(s) to any part or end of the elongate pin. Thus, at least two of the plurality of arms can freely rotate relative to the elongate pin (and its end stop) and the bearing plate. This makes for a low friction join with improved reliability and workability in the hands of a surgeon, as well as lower frictional forces in use. The low friction allows the elongate pin to pass through the apertures of the plurality of arms and rotate with minimal friction, without the need for a rotating bush between the apertures in the plurality of arms and the elongate pin.

The bearing plate may define at least one surface formation that is configured to receive and engage with the deformed region of the end portion of the pin. In some embodiments, at least one surface formation may comprise a rebate or recess formed in a surface of the bearing plate, preferably an outer surface, which faces away from the arms. A rebated well for example advantageously prevents material from the pin flowing onto an arm of the tool as it is deformed. It also allows for the shape of the deformed region of the elongate pin to have a greater thickness, when compared to a bearing plate without a rebate or other recess, which improves the strength of the interference fit. Advantageously, the at least one surface formation may have at least one inner surface which extends in a direction substantially parallel to the pin. For example, a rebated well may define an inner surface that extends circumferentially around the pin. In some embodiments, the bearing plate may comprise one or more apertures configured to receive the elongate pin.

The deformed region of the elongate pin preferably contains no material from the bearing plate. The deformed region may be formed by localised melting and reshaping of the end portion of the elongate pin. The deformed region may be formed by staking, for example formed by heat staking or ultrasonic staking. The deformed region may preferably be formed by ultrasonic staking. Ultrasonic staking provides a low temperature method of forming an interference fit which ensures that the bearing plate is not subjected to temperatures which might cause it to melt. This maintains the mechanical properties of the bearing plate in the tool.

There may be one or more of a stop, washer, film, coating, or low friction filler material located between the end stop and first arm, or between the bearing plate and the second arm.

The plurality of arms and/or elongate pin and/or bearing plate of the surgical tool may be formed from a material that does not conduct electricity. The plurality of arms and/or elongate pin and/or bearing plate of the surgical tool may preferably be formed of a polymer, optionally a carbon-filled polymer, or optionally a glass-filled polymer. A carbon-filled polymer may include carbon fibres (in aligned or random orientation) and/or carbon spheres and/or carbon particles and/or carbon laminate material and/or carbon platelets. A carbon-filled polymer may comprise graphene. A glass-filled polymer may comprise glass fibres (in aligned or random orientation) and/or glass spheres and/or glass particles and/or glass laminate material and/or glass platelets. The glass fibres and/or carbon fibres may be long or short. Alternatively, or additionally to glass fibres or carbon fibres, glass spheres or carbon spheres may be used. A benefit of glass-filled polymer is that it is not electrically conductive (and is, for example, less electrically conductive than carbon-based composites), therefore giving an advantage when used with electrocautery tools. Glass-filled polymers do not conduct heat, which reduces the risk of burning the patient, or the tool itself deforming as a result of the heat. There is also a reduced risk of arcing or other electrical discharge when the tool is used with electrocautery tools, the flashes of such discharges being desirable to avoid, as they may impede the vision of a surgeon using the tool. Optionally the polymer is an amorphous or semi-crystalline polymer suitable for autoclave sterilisation. The polymer may be a thermoplastic synthetic polymer that may optionally be polyether ether ketone (PEEK), nylon 6, nylon 6,6, polyethersulfone (PES), polyoxymethylene (POM or acetal) or a polysulphone polymer.

The deformed region may be formed of thermally reshaped polymer. The end portion may be thermally reshaped to form the interference fit with the bearing plate. The thermal reshaping of the polymer may take the form of localised melting of the end portion of the elongate pin. The end portion of the elongate pin may be reshaped without the melting or thermal deformation of the bearing plate. The thermally reshaped polymer may be formed by ultrasonic staking.

The proximal ends of the first and second arms may together define a handle. The distal ends of the first and second arms may together define a manipulation portion that is configured for use in manipulating a tissue of a body. The manipulation portions may thus be jaw portions. Suitably, the jaw portions may each comprise a plurality of teeth.

The surgical tool may optionally be one of a self-retainer tool, Allis forceps, Babcock forceps, scissors, or another handheld tool.

In the case where the surgical tool is a self-retainer tool, the self-retainer tool may comprise a ratchet device made of a metallic material, optionally stainless steel. At least part of the ratchet device may be mounted to an arm of the surgical tool by way of over-moulding of part of the arm over part of said ratchet device.

In the case where the surgical tool is Allis forceps or Babcock forceps, the tool may comprise a ratchet device located proximal to handle portions of the tool. The ratchet device is optionally integrally formed with the arms of the tool.

In accordance with a second aspect of the present disclosure there is provided a method of manufacturing a surgical tool comprising: providing a plurality of arms, each of the plurality of arms defining an aperture which extends therethrough (at a generally intermediate location), positioning the plurality of arms such that their apertures are mutually aligned, inserting an elongate pin having an end stop at a first end and an elongate portion at an opposite end to the end stop through the aligned apertures of the plurality of arms, such that the end stop is disposed immediately adjacent to a first arm of the plurality of arms; placing a bearing plate on the elongate portion of the pin such that the bearing plate is disposed immediately adjacent to a second arm of the plurality of arms, and deforming the end portion of the elongate pin by staking the end portion of the elongate pin such that the end portion of the elongate pin forms an interference fit with the bearing plate, thereby pivotally joining the plurality of arms relative to each other such that each of the plurality of arms can freely rotate about the elongate pin.

The method step of deforming the end portion of the elongate pin by staking may be performed by ultrasonic staking using a sonotrode configured to be driven to vibrate at ultrasonic frequencies. As mentioned above, ultrasonic staking has advantages as it provides a low temperature method of forming an interference fit which ensures that the bearing plate is not subjected to temperatures which might cause it to melt. This allows the mechanical integrity of the bearing plate to be maintained, thereby improving the performance of the tool. Ultrasonic staking may be performed by ultrasonic staking equipment including a transducer to drive the sonotrode and optionally a booster between said transducer and said sonotrode. A booster may increase the peak-to-peak amplitude of the vibration of the sonotrode.

The plurality of arms and/or the elongate pin and/or the bearing plate of the surgical tool may be formed from a material that does not conduct electricity. The plurality of arms and/or the elongate pin and/or the bearing plate of the surgical tool may be formed of a polymer, optionally a carbon-filled polymer, or optionally a glass-filled polymer. A carbon-filled polymer may include carbon fibres (in aligned or random orientation) and/or carbon spheres and/or carbon particles and/or carbon laminate material and/or carbon platelets. A carbon-filled polymer may comprise graphene. A glass-filled polymer may comprise glass fibres (in aligned or random orientation) and/or glass spheres and/or glass particles and/or glass laminate material and/or glass platelets. A benefit of glass-filled polymer is that it is not electrically conductive (and is, for example, less electrically conductive than carbon-based composites), therefore giving an advantage when used with electrocautery tools. Glass-filled polymers also do not conduct heat, which reduces the risk of burning the patient, or the tool itself deforming due to the heat. There is also a reduced risk of arcing or other electrical discharge when the tool used with electrocautery tools, the flashes of such discharges being desirable to avoid, as they may impede the vision of a surgeon using the tool. Optionally the polymer is an amorphous or semi-crystalline polymer suitable for autoclave sterilisation. The polymer may optionally be a polyether ether ketone (PEEK) or a polysulphone polymer.

The peak-to-peak amplitude of vibration of the sonotrode is optionally between 200 and 500 microns. The peak-to-peak amplitude of vibration of the sonotrode is optionally between 250 and 300 microns. It may be preferable to use a peak-to-peak amplitude within this range as such a range avoids wearing of the sonotrode or fracture of the polymer while still maintaining a sufficiently fast rate of staking.

The sonotrode may be vibrating with a frequency of between 15 kHz and 40 kHz. Optionally, the sonotrode is vibrating with a frequency of between 19.5 kHz and 20.5 kHz. Use of an amplitude in this range may more easily allow for ultrasonic staking using amplitudes above 200 microns. A frequency higher than approximately 40 kHz may reduce the available maximum peak-to-peak amplitude in practice and so may be undesirable.

Optionally, the method step of deforming the end portion of the elongate pin by ultrasonic staking is performed by touching the elongate pin with the sonotrode and applying force to the end portion of the elongate pin using the sonotrode. The sonotrode may be triggered before touching the elongate pin, such that it is vibrating when it first it touches the elongate pin. This may reduce the likelihood of adverse physical jolts or shocks when the sonotrode first touches the elongate pin.

The exposure time of the elongate pin to the sonotrode may be between 100 milliseconds and 2.5 seconds. The force applied to the elongate pin by the sonotrode may be between 5 N and 35 N, preferably between 18 N and 25 N. This aims to ensure deformation occurs at a rate sufficient for the requires production throughput, while not causing damage to any components.

The bearing plate may comprise at least one surface formation that is configured to accommodate the deformed material of elongate pin. The bearing plate may be rebated around a central aperture, preferably in an outer surface of the plate, to accommodate the deformed material of elongate pin. A surface formation, for example, a rebate or recess, serves advantageously to prevent material from the pin flowing onto an arm of the tool as it is deformed, e.g., during staking. It also allows the elongate pin to be formed with an end shape of a greater thickness, as compared to a bearing plate without a rebate or other recess.

Further features and advantages of the methods and surgical tools of the present disclosure will be apparent to those skilled in the art from the following description of various implementations of the disclosure. Thus, following is a description by way of example only with reference to the accompanying drawings of various implementations of the present disclosure.

It will of course be appreciated that features described in relation to one aspect of the present disclosure may be incorporated into other aspects of the present disclosure. For example, the surgical tool according to the first aspect of the disclosure may incorporate any of the features described with reference to the method of manufacturing a surgical tool according to the second aspect of the disclosure, and *vice versa*.

### Brief Description of the Drawings

FIG. 1 is a schematic drawing of a handheld surgical self-retainer forceps in accordance with the present disclosure.
FIG. 2A is a schematic drawing of a handheld surgical Allis forceps in accordance with the present disclosure.
FIG. 2B is side-on view of the handheld surgical forceps shown in FIG. 2A. The view of FIG. 2B is indicated by the viewing direction indicator in FIG 2A.
FIGS. 3A-3D are schematic sectional drawings which show successive steps in the formation of a pin joint between two arms of a handheld surgical instrument in accordance with the present disclosure.
FIG. 4 is a schematic flow diagram of a method of manufacturing a surgical tool in accordance with the present invention.

### Detailed Description

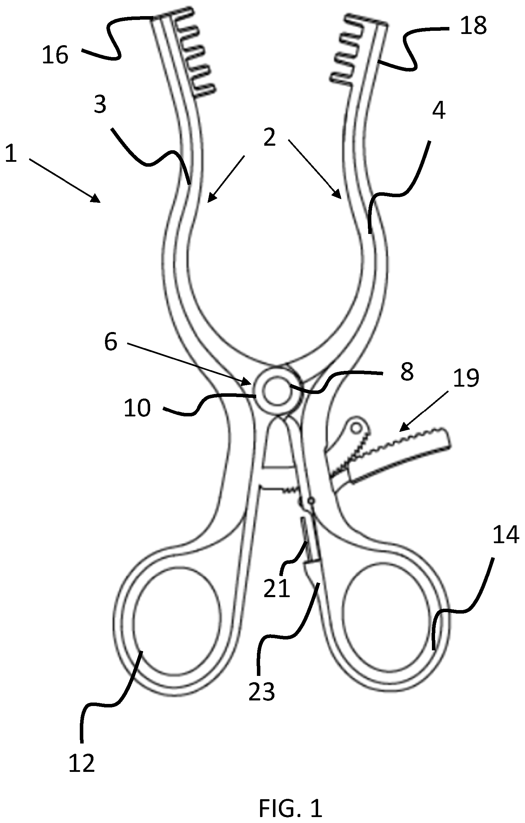

FIG. 1 of the accompanying drawings illustrates schematically a handheld surgical self-retainer (1) in accordance with the present disclosure, when the surgical self-retainer (1) is in an open configuration. As shown in FIG. 1, the self-retainer (1) comprises a pair of arms (2) of which a first arm (3) and a second arm (4) meet at a joint (6) such that they are pivotable about the joint (6) relative to each other. The first arm (3) has an aperture (not shown) that passes through it in a direction transverse to its length, and the second arm (4) has an aperture (not shown) that passes through it in a direction transverse to its length. The joint (6) is made by a pin (8) having a stop (10) at one end of the pin, which is disposed on one side of the pair of arms (2), and a bearing plate (not shown) which is disposed on another opposite side of the pair of arms (2). The bearing plate has a central aperture (not shown) and is arranged such that the pin (8) passes through the aperture. An end portion of the pin (8) is ultrasonically staked to the bearing plate as described in more detail below such that an interference fit is formed between the end of the pin (8) and the bearing plate. The bearing plate is thus fixedly mounted to the pin (8) but is not fastened to either one of the pair of arms (2). Both the first arm (3) and second arm (4) can therefore freely rotate about the pin (8) and the bearing plate. The stop (10) and bearing plate are arranged such that the pair of arms (2) are retained on the pin (8). In this way, the arms (2) are retained immediately adjacent to each other on the pin (8), but excessive inward force or friction is not applied to either of the arms (2), or the pin (8) when the tool is in use.

At a first end of each of the arms (3, 4) is a respective finger ring (12, 14). The finger rings (12, 14) together form a handle. At an opposite end of each arm there is toothed jaw portion (16, 18). The jaw portions (16, 18) together form a pair of jaws that are configured for the gripping and manipulation of human or animal tissue.

The arms (3, 4) and pin (8) of the self-retainer (1) are made of a glass fibre filled polymer. In the present example, the polymer is polyetheretherketone (PEEK).

The self-retainer (1) also comprises a ratchet mechanism (19), and a spring (21) which is configured and arranged to apply force the ratchet mechanism such that the arms (2) can be held in place when the ratchet mechanism (19) is engaged. The ratchet mechanism (19) and spring (21) are made of stainless steel. The spring is mounted to an arm (4) of the self-retainer (1) by an over-moulded section of polymer (23).

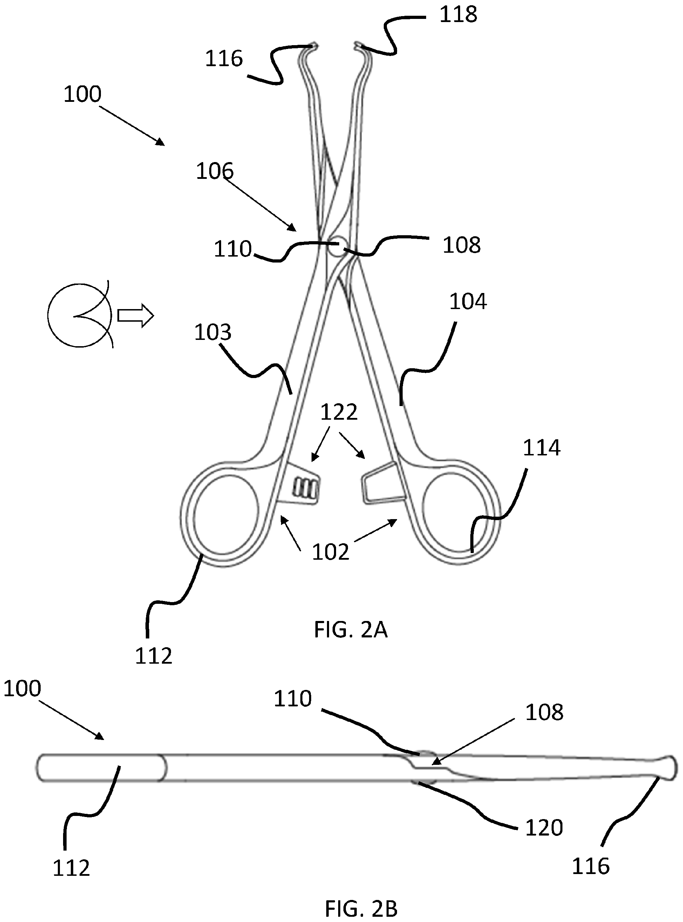

FIGS. 2A and 2B of the accompanying drawings illustrate schematically an Allis forceps in accordance with the present disclosure. As shown in FIG. 2A and FIG. 2B, the Allis forceps (100) has a pair of arms (102) of which a first arm (103) and a second arm (104) meet at a joint (106). The first arm (103) has an aperture (not shown) that passes through it in a direction transverse to its length, and the second arm (104) has an aperture (not shown) that passes through it in a direction transverse to its length. The joint (106) is formed of a pin (108) having a stop (110) at one end, on one side edge of the pair of arms (102) and a bearing plate (120) on the other side of the pair of arms (102). The bearing plate (120) comprises an aperture (not shown) and is arranged such that the pin (108) passes through the aperture. An end of the pin (108) is ultrasonically staked to the bearing plate (120) such that an interference fit it formed between the end of the pin and the bearing plate. Accordingly, the bearing plate (120) is fixedly mounted to the pin (108), but not fixedly mounted to either of the pair of arms (102). Therefore, both the first arm (103) and second arm (104) can freely rotate about the pin (108) and bearing plate (120). The stop (110) and bearing plate (120) are arranged such that the pair of arms (102) are securely retained on the pin (108).

At a first end of each of the pair of arms is a handle (112, 114) and at opposite ends of each arm there are toothed jaws (116, 118) configured for the gripping and manipulation of human tissue.

The arms (102) and elongate pin (108) of the self-retainer are made of a carbon fibre filled polymer. In this example the polymer is a polysulfone polymer.

An integrally formed ratcheted locking mechanism (122) for locking the arms in a number of positions where the jaws (116, 118) are locked at a different position relative to each other is formed in the pair of arms (102) proximal to the handle portions (112, 114) of each of the arms (103, 104).

While the present disclosure has been exemplified herein by reference to a surgical self-retainer, as shown in FIG.1, and an Allis forceps, as shown in FIGS. 2A-2B, those skilled in the art will readily appreciate that the same principles, specifically relating to the manner of joining together two or more (non-metallic) arms with a pin joint, are applicable to a wide range of other types of surgical tools with similar jointed arms such, for example, as a Babcock forceps.

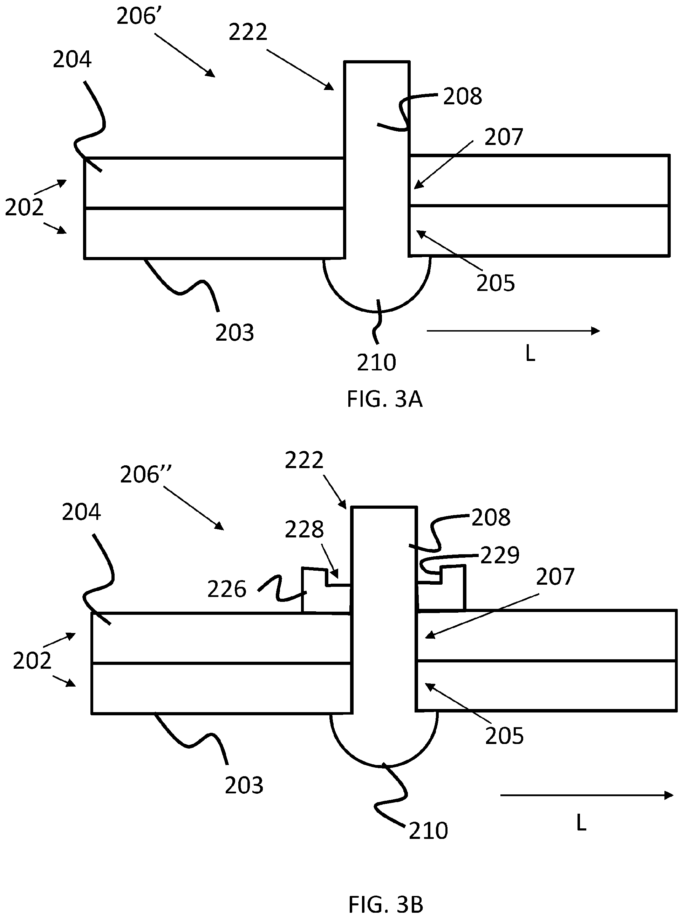

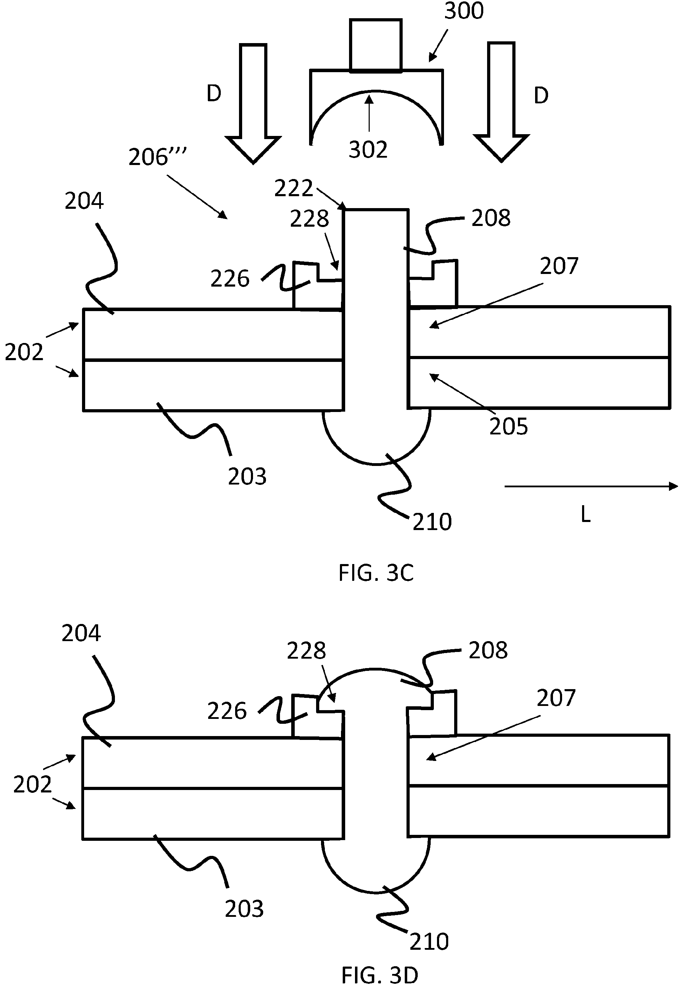

FIGS. 3A, 3B, 3C and 3D illustrate schematically a sectional view of a pin joint (206, 206', 206'', 206‴) which is suitable for use as the joint (6, 106) of the self-retainer (1) of FIG. 1 or the Allis forceps (100) of FIGS. 2A and 2B, or any other similar non-metallic surgical tool comprising jointed arms, at successive stages of assembly, with FIG. 3D showing the joint (206) in its final assembled state.

FIG. 3A shows the pin joint (206') in a first stage of assembly. A pair of elongate arms (202) is provided in which a first arm (203) and a second arm (204) both have an aperture (205, 207) formed therethrough. The apertures (205, 207) pass through the arms in a direction substantially transverse a longitudinal axis (L) of the respective arm. In this first stage of assembly, the apertures (205, 207) of each of the arms (203, 204) are brought into mutual alignment. A pin (208) having an enlarged stop (210) at one end is inserted through the apertures (205, 207). The pin (208) is elongate and has an end portion (222) at an end opposite the stop (210) that extends beyond the second arm of the pair of arms (202). The pin is inserted into the apertures until the stop (210) is contiguous the first arm (203).

FIG. 3B shows the pin joint (206'') in a second stage of assembly. A bearing plate (226) having a central aperture and a rebated well formed in one surface around the aperture is placed over the end portion (222) of the pin (208) such that it is contiguous the second arm (204) with the well facing outwards. The well thus defines a recess (228), which is open in a direction facing away from the second arm (204) towards the end portion (222) of the pin (208). As best shown in FIG. 3B the well includes an inner surface (229) which extends circumferentially around the end portion (222) of the pin (208), substantially parallel to the pin (208).

FIG. 3C shows the joint (206‴) in a third stage of assembly. A sonotrode (300) having a convex tip (302) is moved towards and into abutment with the end portion (222) of the pin (208). The tip (302) of the sonotrode may optionally have a 3-dimensional shape that is substantially the inverse of the shape of the stop (210). The tip (302) of the sonotrode (300) is caused to vibrate with a peak-to-peak amplitude of vibration of about 260 microns. The tip (302) is driven at a vibrational frequency of about 20 kHz, and it is set to begin vibrating before it contacts the end portion (222) of the pin (208).

The process used is an ultrasonic staking process, and accordingly, the sonotrode (300) vibrates while the pin (208), arms (202) and bearing plate (226) are held stationary. This is in sharp contrast to ultrasonic welding, where all components involved in making a joint are arranged to vibrate.

Constant force of about 18 N to 25 N is applied via the sonotrode (300) in direction D as shown in FIG. 3D, as the tip (302) is moved into contact with the end portion (222). The tip (302) is moved in direction D for a total treatment time of about 150 milliseconds. In some implementations of the present disclosure, the sonotrode may be moved in the direction D for a treatment time of up to about 500 milliseconds. With the tip (302) of the sonotrode (300) in contact with the end portion (222) of the pin (208), the end portion is deformed by thermally reshaping the end portion such that the deformed end portion (222) forms an interference fit with the rebated portion of the bearing plate (226), including the inner surface (229) thereof, as shown in Fig. 3D. Note that an interference fit is a physical fit between the bearing plate (226) and the end portion (222) of the elongate pin (208); there is no fusion bonding between pin (208) and the bearing plate (226). This is in sharp contrast to an ultrasonic welding process, which would result in melting and the formation of a fusion bond, via mixing of material, between the pin (208) and the bearing plate (226).

FIG. 3D shows the joint (206) in a fourth stage of assembly, after the ultrasonic staking has finished. The end portion (222) of the pin (208) now has a rounded profile which corresponds to the shape of the tip (302) of the sonotrode (300), as material has deformed into the well (228), thereby taking a shape substantially the same to that of the stop (210). This causes the end (222) of the pin (208) to form a tight physical fit with the well (228). Thus, the bearing plate (226) is secured to the elongate pin (208). The first arm (203) is adjacent but not bonded or fitted to the stop (210) and can move relative to the stop (210) with minimal fiction generated between the first arm (203) and stop (210). The second arm (205) is adjacent but not bonded or fitted to the bearing plate (226) and can move relative to the bearing plate (226) with minimal fiction generated between the second arm (205) and bearing plate (226). The first arm (203) and second arm (205) are therefore secured for free rotation about the elongate pin (208) and cannot slide off the elongate pin (208) because of the presence of the stop (210) and bearing plate (226). This ensures that the arms (202) are held close enough together that any undesirable skewing of the arms (202) is minimised, while ensuring that the forces applied to the arms (202) and joint (206) are minimised such that the wear to the arms (202) and joint (206) is reduced.

The pin joint described with reference to FIGS. 3A-3D and used in the jointed surgical instruments of FIGS. 1, 2A and 2B involves no direct joint between the pin and either of the first and second arms. In this way both arms can rotate freely about the pin. This reduces friction on the arms as compared to known tool designs, thereby improving longevity of the joint. This reduced friction at the joint may also improve the handling of the tools relative to known tool designs. The skilled person would of course appreciate that the above-described pin joint can be extended to other kinds of surgical tools having any number of arms (e.g., 3 or 4 arms).

While not shown in the figures, some implementations of the present disclosure may include one or more of a stop, washer, film, coating, or low friction filler material located between the end stop (210) and first arm (203), or between the bearing plate (226) and the second arm (204), or between the first and second arms (203, 204).

Ultrasonic staking in embodiments in accordance with the present disclosure use an ultrasonic staking equipment stack of the kind well known in the art, which may for example include a transducer (not shown) to drive the tip (302) of the sonotrode (300) and a booster (not shown) between said transducer and said tip to control the amplitude of the tip.

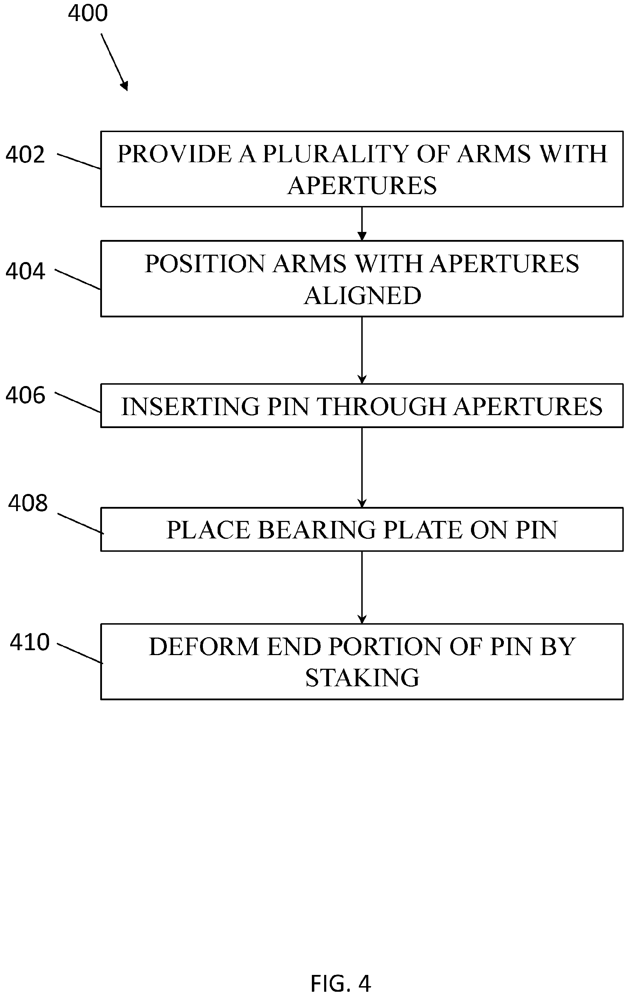

FIG. 4 shows a schematic flow chart depicting a method of manufacturing a surgical tool in accordance with the present disclosure. The method (400) thus includes the successive steps of:
- providing (402) a plurality of arms, each of the plurality of arms having an aperture,
- positioning (404) the plurality of arms such that their apertures are mutually aligned,
- inserting (406) an elongate pin having an end stop at a first end and an elongate portion an opposite end, through the aligned apertures of the plurality of arms, such that the end stop is immediately adjacent to, optionally contiguous, a first arm of the plurality of arms,
- placing (408) a bearing plate on the elongate portion of the pin such that the bearing plate is immediately adjacent to, optionally contiguous, a second arm of the plurality of arms, and
- deforming (410) the end portion of the elongate pin by ultrasonically staking the end portion of the elongate pin such that the end portion of the elongate pin forms an interference fit with the bearing plate, thereby pivotally mounting the plurality of arms relative to each other such that each of the plurality of arms can freely rotate about the elongate pin.

The method is performed by ultrasonic staking using a sonotrode configured to be driven to vibrate at ultrasonic frequencies. The elongate pin and bearing plate of the surgical tool are formed of PEEK. The peak-to-peak amplitude of vibration of the sonotrode is about 230 microns. The sonotrode vibrates with a frequency of about 20 kHz. The bearing plate comprises one or more surface formations such for example as a well or other recess to accommodate the deformed material of elongate pin. The sonotrode is brought into contact with the elongate pin and controlled force is applied to the end portion of the elongate pin using the sonotrode, such that the end portion of the elongate pin deforms and is thermally reshaped such that a mechanical interference fit is formed with the bearing plate, thereby staking the elongate pin to the bearing plate. The sonotrode is triggered before contacting the elongate pin, such that it is vibrating at the point that it first touches the elongate pin. The exposure time of the elongate pin to the sonotrode is about 250ms. The force applied to the sonotrode during the exposure is about 18 N.

In the exemplary implementations described herein, the surgical tools are formed of glass-filled or carbon-filled polymer. The glass-filled or carbon-filled polymer includes or may be filled with glass or carbon fibres. In some implementations, the fibres may be arranged randomly or aligned. The skilled person will however appreciate that the filler may be organised in any known configuration, and may, for example, take the form of a glass laminate or a dispersion of glass/carbon particulates and/or glass/carbon platelets within the polymer. Alternative materials may be used in the construction of the tools, and while such materials are preferably materials that do not conduct electricity, that does not necessarily need to be the case, and tools falling within the scope of the present disclosure may, for example be formed of metal material, such as a steel alloy or a polymer filled with carbon or glass fibre/laminate/particulates/spheres/platelets and/or graphene.

Although aspects of the present disclosure have been described with reference to particular implementations and examples, it is to be understood that these implementations and examples are merely illustrative of the principles and possible applications of the disclosure. It is therefore to be understood that numerous modifications may be made to the illustrative implementations and examples and that other arrangements may be devised without departing from the scope of the disclosure, as defined by the appended claims.

It will be appreciated by those of ordinary skill in the art that features of the implementations and examples may be combined in other implementations that fall within the scope of the present disclosure.

While various details have been set forth in the foregoing description, it will be appreciated that the various aspects of the disclosure may be modified or altered. One skilled in the art will recognise that the herein described specific components are used as examples for the sake of conceptual clarity and that various configuration modifications are contemplated. Consequently, as used herein, the specific exemplars set forth and the accompanying discussion are intended to be representative of their more general classes. In general, use of any specific exemplar is intended to be representative of its class, and the non-inclusion of specific components, devices, and objects should not be taken limiting.

Further, while several forms have been illustrated and described, it is not the intention of the applicant to restrict or limit the scope of the appended claims to such detail. Numerous modifications, variations, changes, substitutions, combinations, and equivalents to those forms may be implemented and will occur to those skilled in the art without departing from the scope of the present disclosure. Moreover, the structure of each element associated with the described forms can be alternatively described as a means for providing the function performed by the element. Also, where materials are disclosed for certain components, other materials may be used. It is therefore to be understood that the foregoing description and the appended claims are intended to cover all such modifications, combinations, and variations as falling within the scope of the disclosed forms. The appended claims are intended to cover all such modifications, variations, changes, substitutions, modifications, and equivalents.

While in the foregoing description, integers or elements are mentioned which have known obvious or foreseeable equivalents, then such equivalents are herein incorporated as if individually set forth. Reference should be made to the claims for determining the true scope of the present disclosure, which should be construed as to encompass any such equivalents. It will also be appreciated by the reader that integers or features of the disclosure that are described as advantageous, suitable, convenient or the like are optional, and do not limit the scope of the independent claims. Moreover, it is to be understood that such optional integers or features, whilst of possible benefit in some embodiments of the disclosure, may not be desirable and may therefore be absent in other embodiments.

When introducing elements of the present disclosure or the preferred embodiment(s) thereof, the articles "a", "an", "the", and "said" are intended to mean that there are one or more of the elements. The terms "comprising", "including", and "having" are intended to be inclusive and mean that there may be additional elements other than the listed elements.

The phrase "and/or," as used herein in the specification and in the claims, should be understood to mean "either or both" of the elements so conjoined, i.e., elements that are conjunctively present in some cases and disjunctively present in other cases.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

The terms "approximately" and "about" may be used to mean within ±20 % of a target value in some embodiments, within ±10 % of a target value in some embodiments, within ±5 % of a target value in some embodiments, and yet within ±2 % of a target value in some embodiments. The terms "approximately" and "about" may include the target value.

In the claims, as well as in the specification above, all transitional phrases such as "comprising," "including," "carrying," "having," "containing," "involving," "holding," "composed of," and the like are to be understood to be open-ended, i.e., to mean including but not limited to. The transitional phrases "consisting of" and "consisting essentially of" shall be closed or semiclosed transitional phrases, respectively.

Where a range or list of values is provided, each intervening value between the upper and lower limits of that range or list of values is individually contemplated and is encompassed within the disclosure as if each value were specifically enumerated herein. In addition, smaller ranges between and including the upper and lower limits of a given range are contemplated and encompassed within the disclosure. The listing of exemplary values or ranges is not a disclaimer of other values or ranges between and including the upper and lower limits of a given range.

The use of headings and sections in the application is not meant to limit the disclosure; each section can apply to any aspect, embodiment, or feature of the disclosure. Only those claims which use the words "means for" are intended to be interpreted under 35 USC 112, sixth paragraph, and even then only in the United States. Absent a recital of "means for" in the claims, such claims should not be construed under 35 USC 112. Outside the United States, the words "means for" are intended to have their natural meaning. Limitations from the specification are not intended to be read into any claims, unless such limitations are expressly included in the claims.

The present invention may be described according to the following numbered clauses:
1. A surgical tool comprising:
   a plurality of arms, wherein each of the plurality of arms defines an aperture;
   an elongate pin extending through the aperture of each of the plurality of arms, said elongate pin having an end stop at a first end of the elongate pin, said end stop being adjacent to the aperture of a first arm of the plurality of arms, and an end portion at a second end of the elongate pin, said end portion extending beyond a second arm of the plurality of arms;
   a bearing plate mounted on said end portion of the pin directly adjacent to the aperture of the second arm, the bearing plate being configured to rotate freely relative to the second arm;
   wherein the end portion comprises a deformed region, which is deformed such that the pin forms an interference fit with the bearing plate, thereby pivotally mounting the plurality of arms relative to each other such that at least one of the plurality of arms can freely rotate about the elongate pin.
2. A surgical tool according to clause 1, wherein at least two of the plurality of arms can freely rotate about the elongate pin.
3. A surgical tool according to clause 1 or clause 2, wherein the bearing plate defines at least one surface formation that is configured to receive and engage with the deformed region of the end portion of the pin.
4. A surgical tool according to any preceding clause wherein the deformed region is formed by ultrasonic staking.
5. A surgical tool according to any preceding clause wherein the elongate pin of the surgical tool is formed of a synthetic polymer, optionally polyether ether ketone (PEEK).
6. A surgical tool according to clause 5 wherein the deformed region is formed of thermally reshaped polymer.
7. A surgical tool according to clause 5 or clause 6, wherein at the elongate pin of the surgical tool is formed of a glass-filled polymer and/or carbon filled polymer, and optionally one or more of: glass fibre filled polymer, carbon fibre filled polymer, glass sphere filled polymer and/or carbon sphere filled polymer.
8. A surgical tool according to any preceding clause wherein proximal ends of each of the plurality of arms together define a handle and distal ends of each of the the plurality of arms together define a manipulation portion that is configured for use in manipulating a tissue of a body.
9. A surgical tool according to any preceding clause, wherein the surgical tool is a self-retainer tool, Allis forceps, Babcock forceps, or scissors.
10. A method of manufacturing a surgical tool comprising:
   a) providing a plurality of arms, each of the plurality of arms having an aperture formed therethrough at a generally intermediate location;
   b) positioning the plurality of arms such that their apertures are mutually aligned,
   c) inserting an elongate pin having an end stop at a first end and an elongate portion at an opposite end to the end stop, through the aligned apertures of the plurality of arms, such that the end stop is disposed immediately adjacent to a first arm of the plurality of arms;
   d) placing a bearing plate on the elongate portion of the pin such that the bearing plate is disposed immediately adjacent to a second arm of the plurality of arms, and
   e) deforming the end portion of the elongate pin by staking the end portion of the elongate pin such that the end portion of the elongate pin forms an interference fit with the bearing plate, thereby pivotally joining the plurality of arms to each other such that each of the plurality of arms can freely rotate about the elongate pin.
11. A method according to clause 10, wherein the step (e) is performed by ultrasonic staking using a sonotrode configured to be driven to vibrate at ultrasonic frequencies.
12. A method according to clause 10 or clause 11, wherein the elongate pin and bearing plate of the surgical tool are formed of polymer, optionally polyether-ether-ketone (PEEK).
13. A method according to clause 11 or clause 12, wherein the peak-to-peak amplitude of vibration of the sonotrode is between 200 and 500 microns, preferably 250 to 300 microns.
14. A method according to any of clauses 11 to 13 wherein the sonotrode vibrates with a frequency of between 15 kHz and 40 kHz, preferably between 19.5 kHz and 20.5 kHz.
15. A method according to any of clauses 11 to 14, wherein the method of step (e) is performed by touching the elongate pin with the sonotrode and applying force to the end portion of the elongate pin using the sonotrode.
16. A method according to clause 15 wherein the sonotrode is triggered before touching the elongate pin, such that it is vibrating when it first touches the elongate pin.
17. A method according to clause 15 or 16, wherein the exposure time of the elongate pin to the sonotrode is between 100 ms and 500 ms.
18. A method according to clause 15 or 17 wherein the force applied to the elongate pin by the sonotrode is between 18 N and 25 N.
19. A method according to any of clauses 10 to 18 wherein the bearing plate comprises at least one surface formation that is configured to accommodate the deformed material of the elongate pin.

## Claims

1. A surgical tool comprising:
a plurality of arms, wherein each of the plurality of arms defines an aperture;
an elongate pin formed of a synthetic polymer, the elongate pin extending through the aperture of each of the plurality of arms, the elongate pin having an end stop at a first end of the elongate pin, the end stop being adjacent to the aperture of a first arm of the plurality of arms, and an end portion at a second end of the elongate pin, the end portion extending beyond a second arm of the plurality of arms;
a bearing plate formed of a polymer, the bearing plate fixedly mounted on the end portion of the pin directly adjacent to the aperture of the second arm, the bearing plate being configured to rotate freely relative to the second arm; and
wherein the end portion comprises a deformed region, deformed such that the pin forms an interference fit with the bearing plate, thereby pivotally mounting the plurality of arms relative to each other such that each of the plurality of arms can freely rotate about the elongate pin and the bearing plate.

2. A surgical tool according to claim 1, wherein the bearing plate is not fixedly mounted to either of the first or second arms.

3. A surgical tool according to claim 1 or claim 2, wherein the bearing plate defines at least one surface formation that is configured to receive and engage with the deformed region of the end portion of the pin.

4. A surgical tool according to claim 3, wherein the at least one surface formation comprises a rebate or recess formed in a surface of the bearing plate, for example a rebated well.

5. A surgical tool according to any preceding claim, wherein the deformed region is formed by ultrasonic staking.

6. A surgical tool according to any preceding claim, wherein the elongate pin is formed of polyether ether ketone (PEEK).

7. A surgical tool according to any preceding claim, wherein the elongate pin is formed of a glass-filled polymer and/or carbon filled polymer, and optionally one or more of: glass fibre filled polymer, carbon fibre filled polymer, glass sphere filled polymer and/or carbon sphere filled polymer.

8. A surgical tool according to any preceding claim wherein proximal ends of each of the plurality of arms together define a handle and distal ends of each of the plurality of arms together define a manipulation portion that is configured for use in manipulating a tissue of a body.

9. A surgical tool according to any preceding claim, wherein the surgical tool is a self-retainer tool, Allis forceps, Babcock forceps, or scissors.

10. A method of manufacturing a surgical tool comprising:
a) providing a plurality of arms, each of the plurality of arms having an aperture formed therethrough at a generally intermediate location;
b) positioning the plurality of arms such that their apertures are mutually aligned;
c) inserting an elongate pin formed of a synthetic polymer and having an end stop at a first end and an elongate portion at an opposite end to the end stop, through the aligned apertures of the plurality of arms, such that the end stop is disposed immediately adjacent to a first arm of the plurality of arms;
d) placing a bearing plate formed of a polymer on the elongate portion of the pin such that the bearing plate is disposed immediately adjacent to a second arm of the plurality of arms; and
e) deforming the end portion of the elongate pin such that the end portion of the elongate pin forms an interference fit with the bearing plate, thereby pivotally joining the plurality of arms to each other such that each of the plurality of arms can freely rotate about the elongate pin and the bearing plate, optionally wherein the deforming is performed by ultrasonic staking using a sonotrode configured to be driven to vibrate at ultrasonic frequencies.

11. A method according to claim 10, wherein the bearing plate is formed of polymer, optionally polyether-ether-ketone (PEEK).

12. A method according to claim 10 or claim 11, wherein the peak-to-peak amplitude of vibration of the sonotrode is between 200 and 500 micrometres, preferably 250 to 300 micrometres.

13. A method according to any of claims 10 to 12, wherein the sonotrode vibrates with a frequency of between 15 kHz and 40 kHz, preferably between 19.5 kHz and 20.5 kHz.

14. A method according to any of claims 10 to 13, wherein the method of step (e) is performed by touching the elongate pin with the sonotrode and applying force to the end portion of the elongate pin using the sonotrode, optionally wherein the sonotrode is triggered before touching the elongate pin, such that it is vibrating when it first touches the elongate pin, optionally wherein the exposure time of the elongate pin to the sonotrode is between 100 ms and 500 ms, optionally wherein the force applied to the elongate pin by the sonotrode is between 18 N and 25 N.

15. A method according to any of claims 10 to 14 wherein the bearing plate comprises at least one surface formation that is configured to accommodate the deformed material of the elongate pin.
